# EUROPEAN PATENT SPECIFICATION

(11) **EP 2 928 527 B1**
(45) Date of publication and mention of the grant of the patent: **13.06.2018**
(21) Application number: 13805805.2
(22) Date of filing: 29.11.2013
(51) Int. Cl.: A61M 5/32

(54) **NEEDLE ASSEMBLY REPLACEMENT DEVICE**
VORRICHTUNG ZUM AUSTAUSCH EINER NADELANORDNUNG
DISPOSITIF DE REMPLACEMENT D'UN ASSEMBLAGE D'AIGUILLE

(30) Priority: 05.12.2012 EP 12195657
(43) Date of publication of application: 14.10.2015
(73) Proprietor: Sanofi-Aventis Deutschland GmbH, 65929 Frankfurt am Main (DE)
(72) Inventor: STEEL, Samuel, Warwick Warwickshire CV31 2TG (GB); DRAPER, Paul Richard, Evesham Worcestershire WR11 3FE (GB); BUTLER, Joseph, Rugby Warwickshire CV21 4DU (GB); CAVE, George, Leamington Spa Warwickshire CV31 1EN (GB); MERCER, David Richard, Leamington Spa Warwickshire CV31 3BY (GB)
(74) Representative: Finger, Catrin
(86) International application number: PCT/EP2013/075133
(87) International publication number: WO 2014/086683

(56) References cited:
- WO-A1-2006/009508
- JP-A- H05 253 300
- US-A- 5 188 598

## Description

### Technical Field

The invention relates to a needle assembly replacement device which is adapted to engage and disengage a needle assembly to/from a medicament delivery device.

### Background of the Invention

Conventional medicament delivery devices typically require a needle assembly for creating a fluid path between a medicament container and a needle. It is generally recommended to use an unused injection needle for each injection in order to reduce the risk for cross contamination, infection and pain associated with reuse of used needles.

Many users of medicament delivery devices, such as pen injectors and autoinjectors, may be elderly or have reduced dexterity. For these groups, mounting needle assemblies to the delivery device may be problematic if the needle assembly is not oriented corrected, which may disrupt the fluid path for the medicament or may lead to a painful injection process. Further, removing used injection needles can be difficult and may subject the user to the risk of needle stick injuries. Generally, it is recommended that needles be removed with care to avoid needle stick injury.

US 5,188,598 A discloses a device for safely removing contaminated needles from syringes without exposing medical personnel to unnecessary health risks. The device requires only one hand to operate; medical personnel simply insert the syringe and press downwardly. The pressure exerted by the user causes disconnect gears to engage the collar of the needle and unscrew the needle from the syringe, at which time the needle falls into a container. The device includes means for forcing needles into the container when the rubber portion of an unscrewed needle gets "trapped" in the disconnect gears, and also includes means for preventing further disengagement of needles when the container cannot safely accommodate additional needles. Also included in the disclosed device is an opening for receiving needles other than the type that can be disconnected by the gears. The container includes a recess for storing a permanent lid, which is applied to the device when the container is full and ready to be disposed.

WO 2006/009508 A1 discloses a device for handling injection needles, especially in relation to injectors, comprising grip means for gripping, removing and retaining a sheath surrounding a needle attached to an injector device, guide means for guiding the sheath back onto the needle after completed injection, means for removing said needle from said injector device and activation means capable of acting on the grip means for removing said needle from said device.

Thus, there remains a need for an improved needle assembly magazine which facilitates needle assembly mounting and removal on/from a medicament delivery device.

### Summary of the Invention

It is an object of the present invention, which is defined in claim 1, to provide a needle assembly replacement device. In an exemplary embodiment, a needle assembly replacement device according to the present invention comprises a chassis, a contact plate translatably disposed on the chassis and having an opening adapted to receive a medicament delivery device, and a plurality of gripping wheels rotatably coupled to the chassis and adapted to releasably engage a cover of a needle assembly. The gripping wheels are adapted to rotate in a first rotational direction when the contact plate translates relative to the chassis in a first axial direction and rotate in a second rotational direction when the contact plate translates relative to the chassis in a second axial direction. Rotation of the gripping wheels in the first rotational direction engages the needle assembly to the medicament delivery device, and rotation of the gripping wheels in the second rotational direction disengages the needle assembly from the medicament delivery device.

In an exemplary embodiment, the opening includes a contoured perimeter adapted to guide the medicament deliver device into a predetermined position relative to the contact plate.

In an exemplary embodiment according to the invention, the needle assembly replacement device further comprises a spring biasing the contact plate in the second axial direction relative to the chassis.

In an exemplary embodiment, the needle assembly replacement device further comprises a plurality of lead screws coupled to the chassis. Each of the lead screws supports one of the gripping wheels. The gripping wheel is adapted to translate and rotate relative to the lead screw. The lead screw includes a helical channel adapted to mate with a radial projection on the gripping wheel.

In an exemplary embodiment, the needle assembly replacement device further comprises a needle support arranged to slide transversely relative to the chassis. The needle support is adapted to hold a new cover of a further needle assembly. The needle support includes a first cam track adapted to receive a first follower pin coupled to a leg which translates in conjunction with the contact plate. Engagement of the first follower pin with the first cam track causes the needle support to slide transversely in a first transverse direction as the contact plate translates in the first axial direction until the new cover is aligned with the opening.

In an exemplary embodiment, the needle assembly replacement device further comprises a needle wedge arranged to slide transversely relative to the chassis. The needle wedge includes a ramp adapted to slide through a through-slot formed in the needle support. The needle wedge includes a second cam track adapted to receive a second follower pin coupled to the leg. Engagement of the second follower pin with the second cam track causes the needle wedge to slide transversely in a first transverse direction as the contact plate translates in the first axial direction until the ramp axially displaces the new cover to engage the gripping wheels.

In an exemplary embodiment, one or more springs bias the needle support and the needle wedge in a second transverse direction opposite the first transverse direction.

In an exemplary embodiment, the second cam track includes a lowermost portion which, when the second follower pin is engaged in the lowermost portion, prevents axial movement of the contact plate in the first axial direction and provides a tactile feedback.

In an exemplary embodiment, the gripping wheels include grooves which, when aligned with the cover, release the cover from the gripping wheels.

The exemplary embodiments of the needle replacement device according to the present invention allow a user to remove a used needle assembly from a medicament delivery device and engage an unused needle assembly to the delivery device without direct contact with either needle assembly. Thus, the risk of needle stick injuries is remarkably reduced.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a schematic perspective view of an exemplary embodiment of a needle assembly replacement device prior to use,
- Figure 2: is a schematic perspective view of an exemplary embodiment of a needle assembly replacement device during use,
- Figure 3: is a schematic detail view of an exemplary embodiment of gripping wheels for a needle assembly replacement device during use,
- Figure 4: is a schematic detail view of an exemplary embodiment of a needle support and a needle wedge for a needle assembly replacement device during use,
- Figure 5: is a schematic perspective view of an exemplary embodiment of a needle assembly replacement device during use,
- Figure 6: is a schematic detail view of an exemplary embodiment of gripping wheels for a needle assembly replacement device during use,
- Figure 7: is a schematic detail view of an exemplary embodiment of a needle support and a needle wedge for a needle assembly replacement device during use,
- Figure 8: is a schematic perspective view of an exemplary embodiment of a needle assembly replacement device during use,
- Figure 9: is a schematic perspective view of an exemplary embodiment of a needle assembly replacement device during use,
- Figure 10: is a schematic detail view of an exemplary embodiment of gripping wheels for a needle assembly replacement device during use,
- Figure 11: is a schematic detail view of an exemplary embodiment of a needle support and a needle wedge for a needle assembly replacement device during use,
- Figure 12: is a schematic perspective view of an exemplary embodiment of a needle assembly replacement device during use,

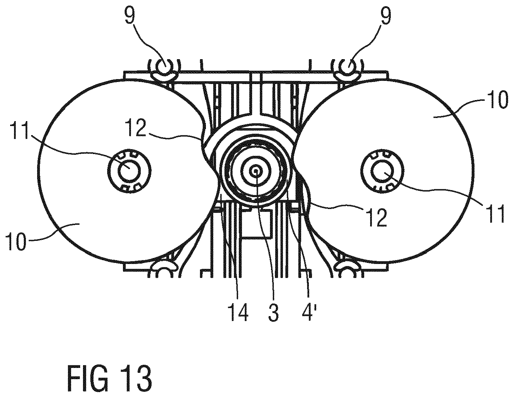

- Figure 13: is a schematic detail view of an exemplary embodiment of gripping wheels for a needle assembly replacement device during use,
- Figure 14: is a schematic perspective view of an exemplary embodiment of a needle assembly replacement device during use,
- Figure 15: is a schematic detail view of an exemplary embodiment of gripping wheels for a needle assembly replacement device during use,
- Figure 16: is a schematic perspective view of an exemplary embodiment of a needle assembly replacement device during use, and
- Figure 17: is a schematic perspective view of an exemplary embodiment of a needle assembly replacement device after use.

Corresponding parts are marked with the same reference symbols in all figures.

### Detailed Description

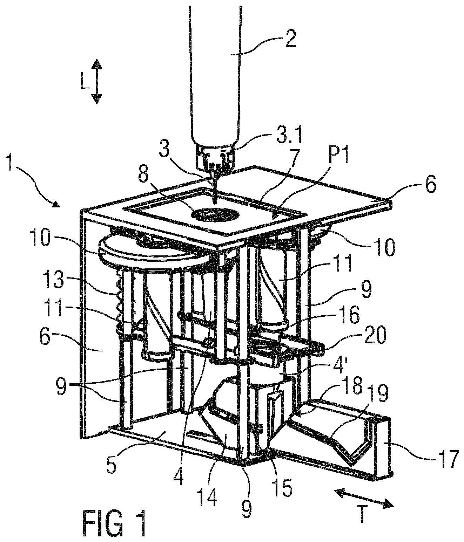

Figure 1 is a schematic perspective view of an exemplary embodiment of a needle assembly replacement device 1 prior to insertion of a medicament delivery device 2. The needle assembly replacement device 1 removes a used needle assembly 3 from a medicament delivery device 2 and engages a new needle assembly 3 to the medicament delivery device 2. In an exemplary embodiment, the needle assembly 3 is attached to the medicament delivery device 2 by a threaded interface, e.g., threads on the needle assembly 3 engage with threads on the delivery device 2.

In an exemplary embodiment, the needle assembly replacement device 1 comprises a chassis 5 with an outer wall 6 and a contact plate 7 having an opening 8 intended to receive a distal end of the medicament delivery device 2 with a needle assembly 3 attached thereto. A portion of the delivery device 2 may contact the contact plate 7 such that axial force applied to the delivery device 2 is transferred to the contact plate 7. The outer wall 6 may partially or wholly enclose the components of the needle assembly replacement device 1. A perimeter of the opening 8 may be contoured to guide the medicament delivery device 2 into a desired position (e.g., perpendicular to the contact plate 7 with a needle assembly 3 attached thereto in the opening 8). For example, a sloped ridge may partially or entirely encircle the opening 8 to guide the medicament delivery device 2 into the opening 8 and ensure that the delivery device 2 remains in the desired position while the needle assembly replacement device 1 is in use. The contact plate 7 is axially moveable relative to the chassis 5 along a number of guiding rails 9 in a longitudinal direction L. A spring 13 is adapted to apply a biasing force on the contact plate 7 relative to the chassis 5.

In an exemplary embodiment, two gripping wheels 10 are coupled to the guiding rails 9 via struts so as to move in the longitudinal direction L with the contact plate 7. Each gripping wheel 10 is coulpled to a lead screw 11 which is fixed to the chassis 5. The lead screw 11 includes a helical channel adapted to mate with a radial projection on an inner circumference of the gripping wheel 10. Thus, as the gripping wheel 10 moves axially relative to the lead screw 11, the engagement of the channel and the radial projection causes the gripping wheel 10 to rotate relative to the lead screw 11. Each gripping wheel 10 may include a frictional surface/material on part or all of its outer circumference.

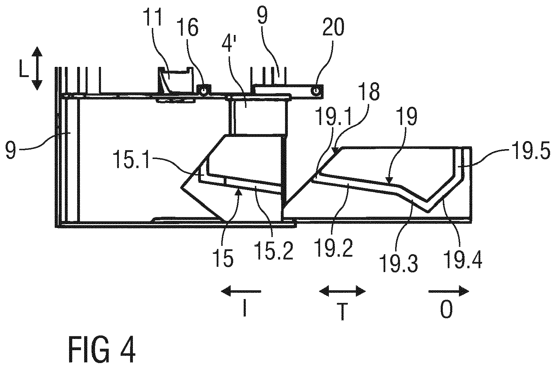

In an exemplary embodiment, a needle support 14 is slidably coupled to the chassis 5 and arranged to slide in a transversal direction T relative to the longitudinal direction L. The needle support 14 is adapted to hold a cover 4' for an unused needle assembly 3. As shown in Figure 4, a first cam track 15 is arranged on the needle support 14 and adapted to receive a first follower pin 16 on a leg connecting two guiding rails 9. The leg is coupled to the contact plate 7 such that axial movement of the contact plate 7 results in axial movement of the leg. The first cam track 15 comprises a longitudinal portion 15.1 parallel with the longitudinal direction L and an angled portion 15.2 having a slope such that the needle support 14 moves in a first transverse direction I on downward movement of the first follower pin 16 when the first follower pin 16 is engaged in the angled portion 15.2.

Referring back to Figure 1, in an exemplary embodiment, a needle wedge 17 having a ramp 18 is slidably coupled to the chassis 5 and arranged to slide in the transversal direction T relative to the longitudinal direction L. The ramp 18 may be arranged to fit within a through-slot in the needle support 14 when the needle wedge 17 moves in the first transversal direction I, and the needle wedge 17 may have a rail which mates with a slot on the needle support 14 (or vice-versa) for aligned translation of the needle wedge 17 relative to the needle support 14. A second cam track 19 is arranged on the needle wedge 17 and is adapted to receive a second follower pin 20 coupled to the leg. As shown in Figure 4, the second cam track 19 comprises a first longitudinal
portion 19.1 in parallel with the longitudinal direction L, a first angled portion 19.2 having a first slope followed by a second angled portion 19.3 having a second slope followed by a third angled portion 19.4 having a third slope followed by a second longitudinal portion 19.5 in parallel with the longitudinal direction L. The slopes of the first angled portion 19.2 and the second angled portion 19.3 are such that the needle wedge 17 moves in the first transversal direction I inwardly on downward movement of the second follower pin 20 when the second follower pin 20 is engaged in the first or second angled portion 19.2, 19.3. The second slope of the second angled portion 19.3 may be steeper then the first slope of the first angled portion 19.2. The third slope of the third angled portion 19.4 may be such that the needle wedge 17 continues movement in the first transversal direction I on upward movement of the second follower pin 20 when the second follower pin 20 is engaged in the third angled portion 19.4.

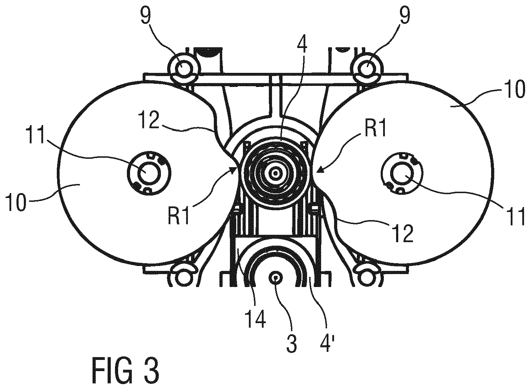

Referring back to Figure 1, the contact plate 7 is in a first axial position P1, pressed against the outer wall 6 under the force of the spring 13. As shown in Figure 3, when the contact plate 7 is in the first axial position P1, the gripping wheels 10 are in a first angular position R1, engaging a cover 4 of the needle assembly 3. In an exemplary embodiment, the gripping wheels 10 are substantially circular with a groove 12 formed in the periphery.

In the exemplary embodiment illustrated in Figure 1, the cover 4 from a used needle assembly 3 is retained by the gripping wheels 10 of the needle assembly replacement device 1, and the used needle assembly 3 is engaged to the delivery device 2.

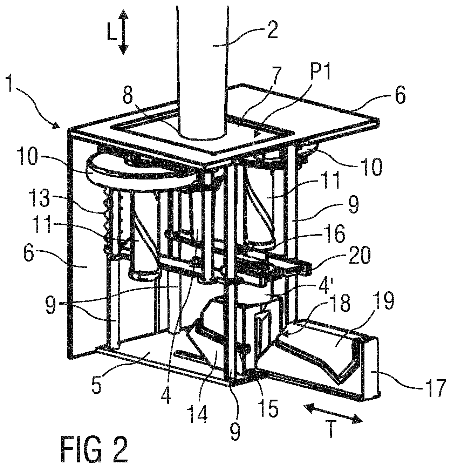

As shown in Figure 2, the distal end of the medicament delivery device 2 with the used needle assembly 3 is inserted in the needle assembly replacement device 1 through the opening 8. The cover 4 may have one or more elements (e.g., ridges, ribs, threads, etc.) which engage corresponding elements on a needle hub 3.1 of the needle assembly 3.

The engagement of the elements and corresponding elements ensure that the needle assembly 3 does not rotate relative to the cover 4.

Figure 4 is a schematic detail view of an exemplary embodiment of the needle support 14 and needle wedge 17 when the contact plate 7 is in the first axial position P1.

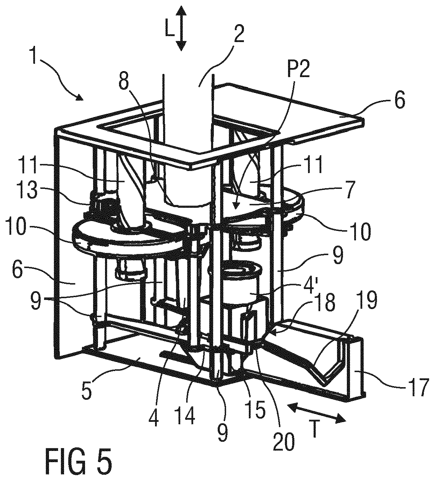

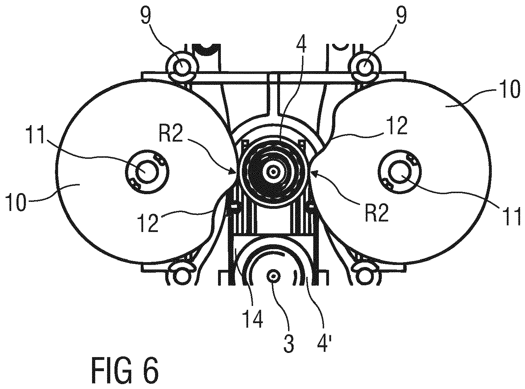

As shown in Figure 5, when an axial force is applied to the medicament delivery device 2, the contact plate 7 is pushed into the chassis 5 to a second axial position P2 against the biasing force of the spring 13. As the contact plate 7 moves axially, the gripping wheels 10 rotate in a first rotational direction due to their interface with the lead screws 11. Rotation of the gripping wheels 10 causes rotation of the cover 4 and the needle hub 3.1 therein relative to the deliver device 2, thus unscrewing the needle assembly 3 from the medicament delivery device 2. When the contact plate 7 is in the second axial position P2, the gripping wheels 10 are in second angular position R2, as shown in Figure 6, maintaining a hold on the cover 4 but having rotated enough to disengage the used needle assembly 3 from the delivery device 2

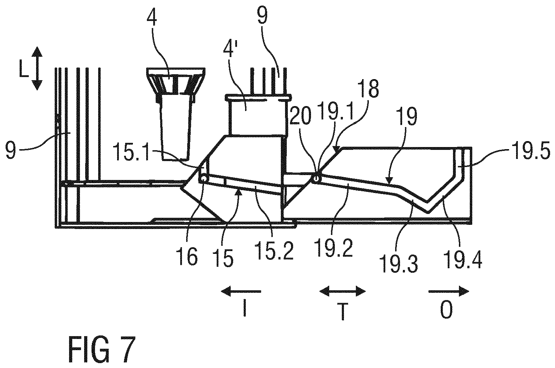

Figure 7 shows the positions of the needle support 14 and needle wedge 17 when the contact plate 7 is in the second axial position P2. The first follower pin 16 has travelled through the longitudinal portion 15.1 of the first cam track 15, and the second follower pin 20 has travelled through the first longitudinal portion 19.1 of the second cam track 19. Because portions 15.1 and 19.1 are parallel to the axis of translation of the contact plate 7, the needle support 14 and the needle wedge 17 do not move when the contact plate 7 moves from the first axial position P1 to the second axial position P2.

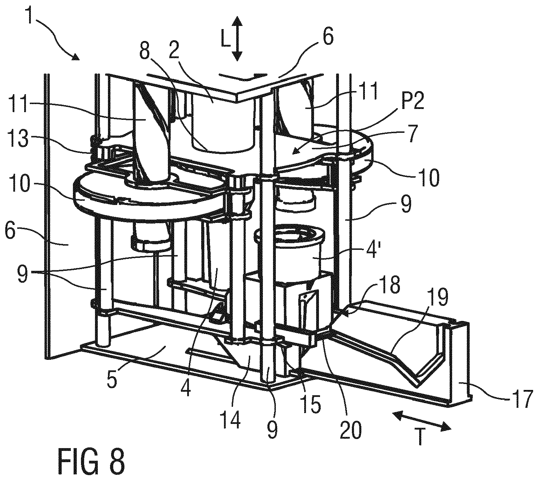

As shown in Figure 8, as the contact plate 7 is pushed into the chassis 5, the cover 4 (with the used needle assembly 3 therein) is rotated in conjunction with rotation of the gripping wheels 10.

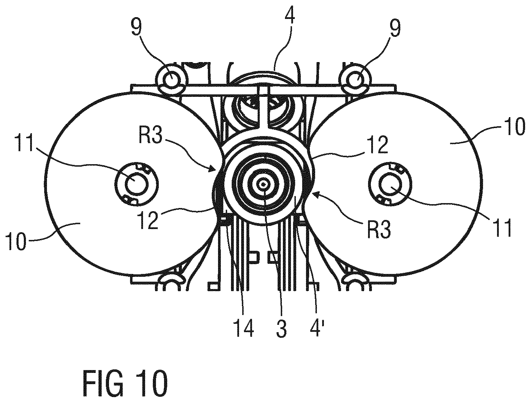

As shown in Figure 10, when the contact plate 7 is in a third axial position P3, the gripping wheels 10 are in a third angular position R3, such that the grooves 12 are aligned with the cover 4, which releases the cover 4 from the gripping wheels 10.

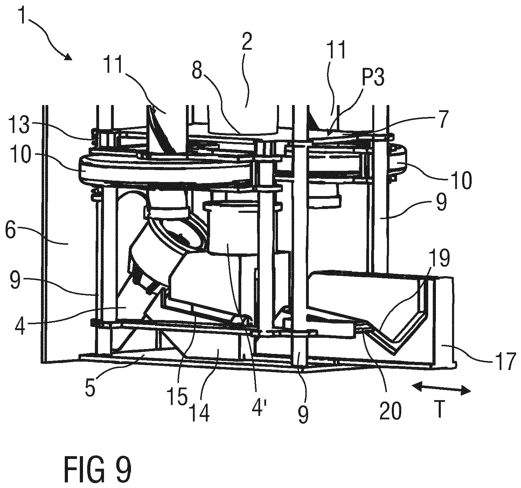

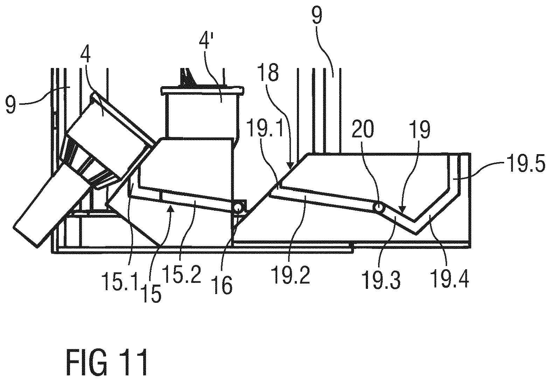

As shown in Figures 9 and 11, when the contact plate 7 is being pushed from second axial position P2 to the third axial position P3, the first follower pin 16 is travelling through the angled portion 15.2 of the first cam track 15 in the needle support 14 such that needle support 14 is moved in the first transversal direction I, and the second follower pin 20 is travelling through the first angled portion 19.2 of the second cam track 19 in the needle wedge 17 such that needle wedge 17 is moved in the first transversal direction I. Thus, when the cover 4 (and the used needle assembly 3 therein) is released from the gripping wheels 10, a ramp 22 on the needle support 14 abuts the cover 4 and pushes it in the first transversal direction I through an opening 21 in the wall 6. In an exemplary embodiment, the slope of the angled portion 15.2 of the first cam track 15 substantially equals the first slope of the first angled portion 19.2 of the second cam track 19 such that the amount of transversal travel of the needle support 14 and the needle wedge 17 is equal once the first follower pin 16 is engaged in the angled portion 15.2 and the second follower pin 20 is engaged in the first angled portion 19.2. When the contact plate 7 is in the third axial position P3, the first follower pin 16 has exited the first cam track 15, and a new cover 4' containing an unused needle assembly 3 is aligned with the opening 8 and the distal end of the delivery device 2. Further, because the first follower pin 16 has exited the first cam track 15, continued translation of the contact plate 7 does not cause any movement of the needle support 14.

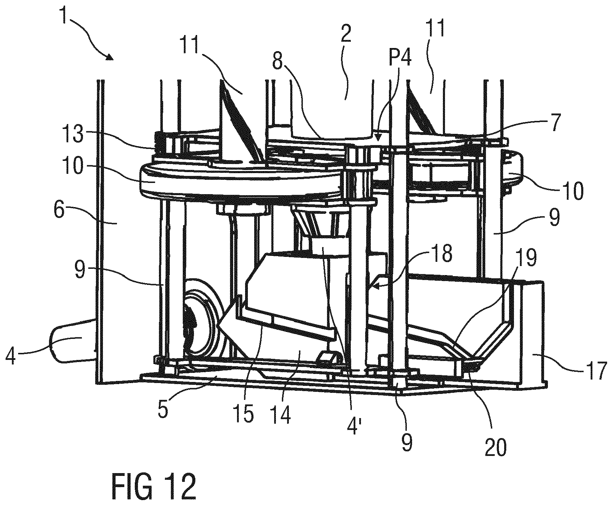

As shown in Figure 12, when the contact plate 7 is pushed to a fourth axial position P4, the needle wedge 17 moves in the first transversal direction I, and the ramp 18 on the needle wedge 17 enters the slot of the needle support 14 and pushes the new cover 4' upwards towards the distal end of the medicament delivery device 2 to a level at which the gripping wheels 10 can interface with the new cover 4'. In the fourth axial position P4, the second follower pin 20 has reached a lower-most portion of the second cam track 19, preventing further downward movement of the contact plate 7 and providing a tactile feedback such that force on the delivery device 2 can be released or reduced. When the contact plate 7 is in the fourth axial position P4, the gripping wheels 10 do not sufficiently engage the new cover 4'.

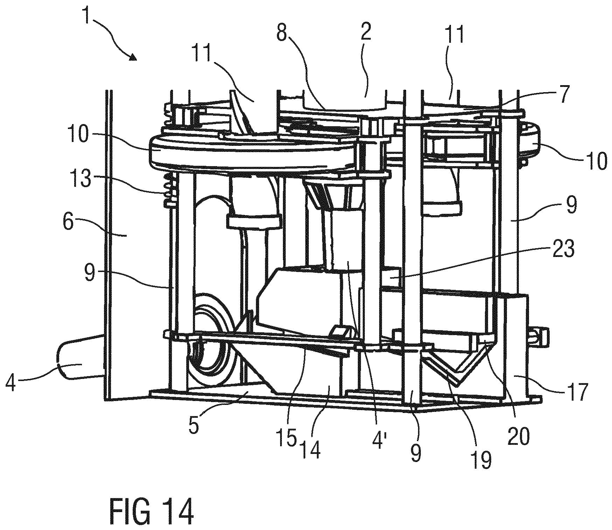

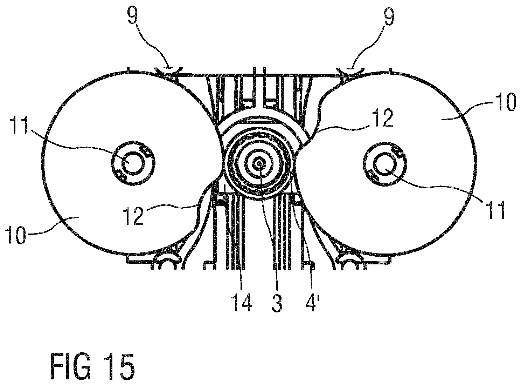

As shown in Figure 14, when the force on the delivery device 2 is reduced or released, the biasing force in the spring 13 pushes the contact plate 7. As the contact plate 7 translates axially, the second follower pin 20 is engaged in the third angled portion 19.4, causing the needle wedge 17 to move further in the first transversal direction I and have the ramp 18 push the new cover 4' (and unused needle assembly 3 therein) upward matching the upward travel of the contact plate 7 and medicament delivery device 2. During this movement, the gripping wheels 10 rotate in a second rotational direction opposed to the first rotational direction and grip (as shown in Figure 15) the new cover 4' before the axial support by the needle wedge 17 is removed when the second follower pin 20 reaches the second longitudinal portion 19.5 of the second cam track 19 in the needle wedge 17.

In an exemplary embodiment, the needle support 14 and the needle wedge 17 are biased in a second transversal direction O by one or more springs (not shown). As the first follower pin 16 abuts a lateral face 23 of the needle support 14 opposite the ramp 22 and the second follower pin 20 is still engaged in the second longitudinal portion 19.5 of the second track 19 in the needle wedge 17, the needle support 14 and the needle wedge 17 are prevented from moving in the second transversal direction O until the contact plate 7 has travelled upward by a sufficient distance to allow the follower pins 20, 16 to disengage the needle wedge 17 and needle support 14. This is to ensure that the new cover 4' is clear of the needle support 14.

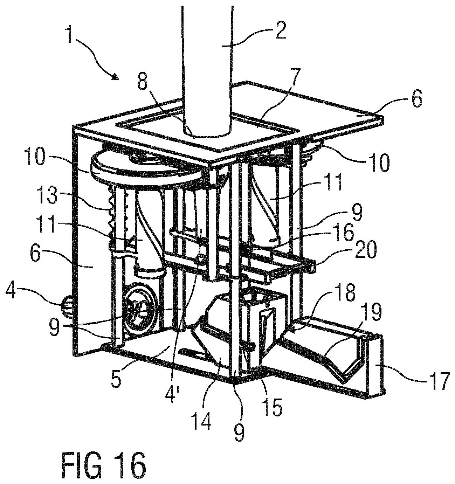

As the medicament delivery device 2 and the contact plate 7 move further upward out of the chassis 5, the thread interface between the gripping wheels 10 and the lead screws 11 cause the gripping wheels 10 to rotate in the second rotational direction. This rotates the new cover 4' (and hence unused needle assembly 3 therein) for engaging the unused needle assembly 3 to the medicament delivery device , as shown in Figure 16.

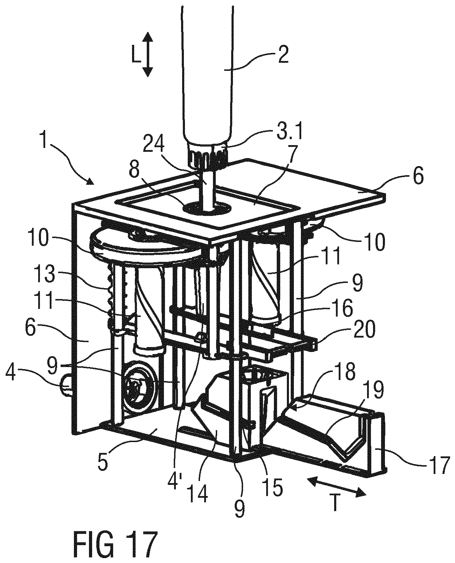

Figure 17 is a schematic perspective view of the needle assembly replacement device 1 during removal of the medicament delivery device 2. An inner needle cover 24 is still attached to the needle assembly 3. The presence of the cover 24 may provide a visual feedback that an unused needle assembly 3 has been assembled to the medicament delivery device 2. The new cover 4' remains in the needle assembly replacement device 1.

In alternative exemplary embodiments, the needle support 14 may be arranged to retain more than one new needle assembly 3 in respective new covers 4'.
Those of skill in the art will understand that modifications (additions and/or removals) of various components of the apparatuses, methods and/or systems and embodiments described herein may be made without departing from the full scope of the present invention, which is defined in the appended claims.

## Claims

1. A needle assembly replacement device (1) comprising:
a chassis (5)
a contact plate (7) translatably disposed on the chassis (5) and having an opening (8) adapted to receive a medicament delivery device (2); and
a plurality of gripping wheels (10) rotatably coupled to the chassis (5) and adapted to releasably engage a cover (4) of a needle assembly (3), the cover (4) having one or more elements engaging corresponding elements on a needle hub (3.1) of the needle assembly (3) for preventing the needle assembly (3) rotating relative to the cover (4), the gripping wheels (10) adapted to rotate in a first rotational direction when the contact plate (7) translates into the chassis (5) in a first axial direction against the biasing force of a spring (13) and rotate in a second rotational direction when the contact plate (7) translates relative to the chassis (5) in a second axial direction pushed by the biasing force of the spring (13), and
wherein rotation of the gripping wheels (10) in the first rotational direction unscrews the needle assembly (3) from the medicament delivery device (2), and rotation of the gripping wheels (10) in the second rotational direction engages an unused needle assembly (3) to the medicament delivery device (2) .

2. The needle assembly replacement device (1) according to claim 1, wherein the opening (8) includes a contoured perimeter adapted to guide the medicament delivery device (2) into a predetermined position relative to the contact plate (7).

3. The needle assembly replacement device (1) according to any one of the preceding claims, further comprising:
a plurality of lead screws (11) coupled to the chassis (5), wherein each of the lead screws (11) supports one of the gripping wheels (10), and wherein the gripping wheel (10) is adapted to translate and rotate relative to the lead screw (11).

4. The needle assembly replacement device (1) according to claim 3, wherein the lead screw (11) includes a helical channel adapted to mate with a radial projection on the gripping wheel (10).

5. The needle assembly replacement device (1) according to any one of the preceding claims, further comprising:
a needle support (14) arranged to slide transversely relative to the chassis (5), wherein the needle support (14) is adapted to hold a new cover (4') of a further needle assembly.

6. The needle assembly replacement device (1) according to claim 5, wherein the needle support (14) includes a first cam track (15) adapted to receive a first follower pin (16) coupled to a leg which translates in conjunction with the contact plate (7).

7. The needle assembly replacement device (1) according to claim 6, wherein engagement of the first follower pin (16) with the first cam track (15) causes the needle support (14) to slide transversely in a first transverse direction (I) as the contact plate (7) translates in the first axial direction until the new cover (4') is aligned with the opening (8).

8. The needle assembly replacement device (1) according to claims 5, 6, or 7, further comprising:
a needle wedge (17) arranged to slide transversely relative to the chassis (5), wherein the needle wedge (17) includes a ramp (18) adapted to slide through a through-slot formed in the needle support (14).

9. The needle assembly replacement device (1) according to claim 8, wherein the needle wedge (17) includes a second cam track (19) adapted to receive a second follower pin (20) coupled to the leg.

10. The needle assembly replacement device (1) according to claim 9, wherein engagement of the second follower pin (20) with the second cam track (19) causes the needle wedge (17) to slide transversely in a first transverse direction (I) as the contact plate (7) translates in the first axial direction until the ramp (18) axially displaces the new cover (4') to engage the gripping wheels (10).

11. The needle assembly replacement device (1) according to claims 7 and 10, wherein one or more springs bias the needle support (14) and the needle wedge (17) in a second transverse direction (O) opposite the first transverse direction (I).

12. The needle assembly replacement device (1) according to claim 10, wherein the second cam track (19) includes a lowermost portion which, when the second follower pin (20) is engaged in the lowermost portion, prevents axial movement of the contact plate (7) in the first axial direction and provides a tactile feedback.

13. The needle assembly replacement device (1) according to any one of the preceding claims, wherein the gripping wheels (10) include grooves (12) which, when aligned with the cover (4), release the cover (4) from the gripping wheels (10).

## Patentansprüche

1. Vorrichtung (1) zum Austausch einer Nadelanordnung, umfassend:
ein Chassis (5),
eine Kontaktplatte (7), die verschiebbar auf dem Chassis (5) angeordnet ist und eine Öffnung (8) aufweist, die zur Aufnahme einer Medikamenten-Verabreichungsvorrichtung (2) ausgelegt ist; und
eine Mehrzahl von Greifrädern (10), die drehbar mit dem Chassis (5) verbunden sind und die dazu ausgelegt sind, eine Abdeckung (4) einer Nadelanordnung (3) freigebbar in Eingriff zu nehmen, wobei die Abdeckung (4) ein oder mehrere Elemente aufweist, die entsprechende Elemente auf einem Nadelansatz (3.1) der Nadelanordnung (3) in Eingriff nehmen, um eine Drehung der Nadelanordnung (3) bezüglich der Abdeckung (4) zu verhindern, wobei die Greifräder (10) dazu ausgelegt sind, sich in einer ersten Drehrichtung zu drehen, wenn sich die Kontaktplatte (7) in einer ersten axialen Richtung gegen die Vorspannkraft einer Feder (13) in das Chassis (5) verschiebt, und sich in einer zweiten Drehrichtung zu drehen, wenn sich die Kontaktplatte (7) bezüglich des Chassis (5) in einer zweiten axialen Richtung, geschoben durch die Vorspannkraft der Feder (13), verschiebt, und
wobei eine Drehung der Greifräder (10) in der ersten Drehrichtung die Nadelanordnung (3) von der Medikamenten-Verabreichungsvorrichtung (2) abschraubt, und eine Drehung der Greifräder (10) in der zweiten Drehrichtung eine unbenutzte Nadelanordnung (3) mit der Medikamenten-Verabreichungsvorrichtung (2) in Eingriff bringt.

2. Vorrichtung (1) zum Austausch einer Nadelanordnung nach Anspruch 1, wobei die Öffnung (8) einen konturierten Umfang aufweist, der dazu ausgelegt ist, die Medikamenten-Verabreichungsvorrichtung (2) in eine vorbestimmte Stellung bezüglich der Kontaktplatte (7) zu führen.

3. Vorrichtung (1) zum Austausch einer Nadelanordnung nach einem der vorhergehenden Ansprüche, ferner umfassend:
eine Mehrzahl von Leitspindeln (11), die mit dem Chassis (5) verbunden sind, wobei jede der Leitspindeln (11) eines der Greifräder (10) unterstützt, und wobei das Greifrad (10) dazu ausgelegt ist, sich bezüglich der Leitspindel (11) zu verschieben und zu drehen.

4. Vorrichtung (1) zum Austausch einer Nadelanordnung nach Anspruch 3, wobei die Leitspindel (11) einen spiralförmigen Kanal aufweist, der dazu ausgelegt ist, mit einem radialen Vorsprung auf dem Greifrad (10) zusammenzupassen.

5. Vorrichtung (1) zum Austausch einer Nadelanordnung nach einem der vorhergehenden Ansprüche, ferner umfassend:
einen Nadelträger (14), der dazu angeordnet ist, bezüglich des Chassis (5) quer zu gleiten, wobei der Nadelträger (14) dazu ausgelegt ist, eine neue Abdeckung (4') einer weiteren Nadelanordnung zu halten.

6. Vorrichtung (1) zum Austausch einer Nadelanordnung nach Anspruch 5, wobei der Nadelträger (14) eine erste Nockenbahn (15) aufweist, die zur Aufnahme eines ersten Mitnehmerstifts (16) ausgelegt ist, der mit einem Schenkel verbunden ist, der sich zusammen mit der Kontaktplatte (7) verschiebt.

7. Vorrichtung (1) zum Austausch einer Nadelanordnung nach Anspruch 6, wobei ein Eingriff des ersten Mitnehmerstifts (16) mit der ersten Nockenbahn (15) verursacht, dass der Nadelträger (14) quer in eine erste Querrichtung (I) gleitet, wenn sich die Kontaktplatte (7) in die erste axiale Richtung verschiebt, bis die neue Abdeckung (4') mit der Öffnung (8) ausgerichtet ist.

8. Vorrichtung (1) zum Austausch einer Nadelanordnung nach den Ansprüchen 5, 6 oder 7, ferner umfassend:
einen Nadelkeil (17), der dazu angeordnet ist, bezüglich des Chassis (5) quer zu gleiten, wobei der Nadelkeil (17) eine Rampe (18) aufweist, die dazu ausgelegt ist, durch einen im Nadelträger (14) ausgebildeten Durchgangsschlitz zu gleiten.

9. Vorrichtung (1) zum Austausch einer Nadelanordnung nach Anspruch 8, wobei der Nadelkeil (17) eine zweite Nockenbahn (19) aufweist, die zur Aufnahme eines zweiten, mit dem Schenkel verbundenen Mitnehmerstifts (20) ausgelegt ist.

10. Vorrichtung (1) zum Austausch einer Nadelanordnung nach Anspruch 9, wobei ein Eingriff des zweiten Mitnehmerstifts (20) mit der zweiten Nockenbahn (19) verursacht, dass der Nadelkeil (17) quer in einer ersten Querrichtung (I) gleitet, wenn sich die Kontaktplatte (7) in die erste axiale Richtung verschiebt, bis die Rampe (18) die neue Abdeckung (4') axial verdrängt, um die Greifräder (10) in Eingriff zu nehmen.

11. Vorrichtung (1) zum Austausch einer Nadelanordnung nach den Ansprüchen 7 und 10, wobei eine oder mehrere Federn den Nadelträger (14) und den Nadelkeil (17) in eine zweite Querrichtung (O), die der ersten Querrichtung (I) entgegengesetzt ist, vorspannen.

12. Vorrichtung (1) zum Austausch einer Nadelanordnung nach Anspruch 10, wobei die zweite Nockenbahn (19) einen untersten Abschnitt aufweist, der, wenn der zweite Mitnehmerstift (20) mit dem untersten Abschnitt in Eingriff ist, eine axiale Bewegung der Kontaktplatte (7) in die erste axiale Richtung verhindert und eine taktile Rückmeldung liefert.

13. Vorrichtung (1) zum Austausch einer Nadelanordnung nach einem der vorhergehenden Ansprüche, wobei die Greifräder (10) Nuten (12) aufweisen, die, wenn sie mit der Abdeckung (4) ausgerichtet sind, die Abdeckung (4) von den Greifrädern (10) lösen.

## Revendications

1. Dispositif (1) de remplacement d'un ensemble aiguille, comprenant :
un châssis (5) ;
une plaque de contact (7) disposée à translation sur le châssis (5) et comportant une ouverture (8) apte à recevoir un dispositif (2) d'administration de médicament ; et
une pluralité de roues de préhension (10) accouplées en rotation au châssis (5) et aptes à entrer en prise amovible avec un couvercle (4) d'un ensemble aiguille (3), le couvercle (4) comportant un ou plusieurs éléments entrant en prise avec des éléments correspondants sur un pavillon (3.1) d'aiguille de l'ensemble aiguille (3) pour empêcher l'ensemble aiguille (3) de tourner par rapport au couvercle (4), les roues de préhension (10) étant aptes à tourner dans un premier sens de rotation quand la plaque de contact (7) pénètre dans le châssis (5) par translation dans une première direction axiale en surmontant la force de sollicitation d'un ressort (13) et tourner dans un second sens de rotation quand la plaque de contact (7) effectue une translation par rapport au châssis (5) dans une seconde direction axiale sous la poussée de la force de sollicitation du ressort (13), et
dans lequel la rotation des roues de préhension (10) dans le premier sens de rotation dévisse l'ensemble aiguille (3) du dispositif (2) d'administration de médicament, et la rotation des roues de préhension (10) dans le second sens de rotation met un ensemble aiguille (3) n'ayant pas servi en prise avec le dispositif (2) d'administration de médicament.

2. Dispositif (1) de remplacement d'un ensemble aiguille selon la revendication 1, dans lequel l'ouverture (8) comprend un périmètre profilé apte à guider le dispositif (2) d'administration de médicament vers une position prédéterminée par rapport à la plaque de contact (7).

3. Dispositif (1) de remplacement d'un ensemble aiguille selon l'une quelconque des revendications précédentes, comprenant en outre une pluralité de broches filetées (11) accouplées au châssis (5), dans lequel chacune des broches filetées (11) soutient l'une des roues de préhension (10), et dans lequel la roue de préhension (10) est apte à se déplacer en translation et rotation par rapport à la broche filetée (11).

4. Dispositif (1) de remplacement d'un ensemble aiguille selon la revendication 3, dans lequel la broche filetée (11) comprend un canal hélicoïdal apte à s'accoupler avec une saillie radiale sur la roue de préhension (10).

5. Dispositif (1) de remplacement d'un ensemble aiguille selon l'une quelconque des revendications précédentes, comprenant en outre un support (14) d'aiguille agencé pour coulisser transversalement par rapport au châssis (5),
dans lequel le support (14) d'aiguille est apte à maintenir un nouveau couvercle (4') d'un autre ensemble aiguille.

6. Dispositif (1) de remplacement d'un ensemble aiguille selon la revendication 5, dans lequel le support (14) d'aiguille comprend une première rampe de guidage (15) apte à recevoir une première broche suiveuse (16) accouplée à une patte qui effectue une translation conjointe avec la plaque de contact (7).

7. Dispositif (1) de remplacement d'un ensemble aiguille selon la revendication 6, dans lequel l'entrée en prise de la première broche suiveuse (16) avec la première rampe de guidage (15) fait coulisser transversalement le support (14) d'aiguille dans une première direction transversale (I) lorsque la plaque de contact (7) effectue une translation dans la première direction axiale jusqu'à ce que le nouveau couvercle (4') soit aligné avec l'ouverture (8).

8. Dispositif (1) de remplacement d'un ensemble aiguille selon la revendication 5, 6 ou 7, comprenant en outre une cale (17) d'aiguille agencée pour coulisser transversalement par rapport au châssis (5),
dans lequel la cale (17) d'aiguille comprend une rampe (18) apte à coulisser à travers une fente traversante pratiquée dans le support (14) d'aiguille.

9. Dispositif (1) de remplacement d'un ensemble aiguille selon la revendication 8, dans lequel la cale (17) d'aiguille comprend une seconde rampe de guidage (19) apte à recevoir une seconde broche suiveuse (20) accouplée à la patte.

10. Dispositif (1) de remplacement d'un ensemble aiguille selon la revendication 9, dans lequel l'entrée en prise de la seconde broche suiveuse (20) avec la seconde rampe de guidage (19) fait coulisser transversalement la cale (17) d'aiguille dans une première direction transversale (I) lorsque la plaque de contact (7) effectue une translation dans la première direction axiale jusqu'à ce que la rampe (18) déplace axialement le nouveau couvercle (4') pour qu'il entre en prise avec les roues de préhension (10).

11. Dispositif (1) de remplacement d'un ensemble aiguille selon les revendications 7 et 10, dans lequel un ou plusieurs ressorts sollicitent le support (14) d'aiguille et la cale (17) d'aiguille dans une seconde direction transversale (O) opposée à la première direction transversale (I).

12. Dispositif (1) de remplacement d'un ensemble aiguille selon la revendication 10, dans lequel la seconde rampe de guidage (19) comprend une partie la plus basse qui, quand la seconde broche suiveuse (20) s'engage dans la partie la plus basse, empêche le déplacement axial de la plaque de contact (7) dans la première direction axiale et fournit une réaction tactile.

13. Dispositif (1) de remplacement d'un ensemble aiguille selon l'une quelconque des revendications précédentes, dans lequel les roues de préhension (10) comprennent des rainures (12) qui, quand elles sont alignées avec le couvercle (4), libèrent le couvercle (4) des roues de préhension (10).
